# NEUE EUROPÄISCHE PATENTSCHRIFT

(11) **EP 0 806 198 B2**
(45) Veröffentlichungstag und Bekanntmachung des Hinweises auf die Entscheidung über den Einspruch: **12.09.2007**
(45) Hinweis auf die Patenterteilung: 21.04.2004
(21) Anmeldenummer: 97104473.0
(22) Anmeldetag: 15.03.1997
(51) Int. Cl.: A61Q 5/10, A61K 8/00, A61K 8/36, A61K 8/37, A61K 8/38, A61K 8/40, A61K 8/41, A61K 8/46, A61K 8/49, A61K 8/60, A61K 8/365

(54) **Färbemittel**
Dyeing composition
Composition de teinture

(30) Priorität: 09.05.1996 DE 19618595
(43) Veröffentlichungstag der Anmeldung: 12.11.1997
(73) Patentinhaber: Wella Aktiengesellschaft, 64274 Darmstadt (DE)
(72) Erfinder: Kunz, Manuela, Dr., 1723 Marly (CH); Le Cruer, Dominique, 1723 Marly (CH); Balzer, Wolfgang R., Dr., 64665 Alsbach (DE)

(56) Entgegenhaltungen:
- DE-A- 2 631 318
- DE-A- 3 115 537
- DE-B- 2 932 489
- US-A- 3 506 990
- US-A- 3 630 654
- US-A- 3 836 327
- US-A- 4 192 648
- CHEMICAL ABSTRACTS, vol. 67, no. 8, 21. August 1967 (1967-08-21) Columbus, Ohio, US; abstract no. 33749, CSUROS, ZOLTAN ET AL: "Dyeing of polyester fibers in the presence of carriers" XP002144201 & PRZEGL. WLOK. (1967), 21(2), 90-1,

## Beschreibung

Die vorliegende Erfindung betrifft ein Mittel zum Färben von Fasern unter Verwendung einer Kombination aus Farbstoffen mit mindestens einer sauren Gruppe und Carriern. Als Fasermaterial kommen hierbei sowohl natürliche Fasern, insbesondere menschliche Haare, als auch synthetische Fasern in Betracht.

In Haarfärbemitteln auf der Basis von direktziehenden Farbstoffen werden insbesondere kationische und neutrale Farbstoffe eingesetzt. Hierbei können die in der Regel sehr kleinen neutralen Farbstoffe relativ gut in das Haarkeratin eindringen, während sich die in der Regel deutlich größeren basischen Farbstoffe an die sauren Gruppen der Haaroberfläche anlagern, so daß relativ waschbeständige salzartige Bindungen entstehen.

Farbstoffe mit sauren Gruppen finden ausgedehnte Anwendung in der Textilfärbung. Eine bedeutende Gruppe bilden hier insbesondere die Azofarbstoffe. Einige der sauren Farbstoffe sind auch in der Nahrungsmittelindustrie zum Anfärben von Lebensmitteln zugelassen.

Vergleicht man diese sauren Farbstoffe mit den in direktziehenden Haarfärbemitteln normalerweise einge-setzten basischen oder neutralen Farbstoffen, so fällt auf, daß es sich häufig um relativ große Moleküle mit einer oder mehreren negativen Ladungen handelt. Da das Haarkeratin, wenn überhaupt, negativ geladen ist, kann es zur Abstoßung des ebenfalls negativ geladenen Farbstoffes kommen.

Werden Haare mit basischen (anionischen) Farbstoffen im sauren pH-Bereich gefärbt, so erhält man lediglich relativ schwache und wenig waschbeständige Färbungen. So ergibt zum Beispiel eine dunkelbraune Färbemasse eine orange-braune Färbung.

Aus der US-A-3 630 654 sind Haarfärbemittel bekannt, welche einen sauren Farbstoff, ein wasserlösliches Polymer mit freien Carboxylatgruppen und ein Lösungsmittel enthalten, wobei als geeignete Lösungsmittel unter anderem Alkohole, wie zum Beispiel Benzylalkohol oder Phenylpropanol, genannt werden.
In der US-A-3 836 327 wird ein Verfahren zum Färben von aromatischen Polyamidfasern beschrieben, bei dem aromatische Alkohole, wie zum Beispiel Benzylalkohol, Tolylalkohole oder 2-Phenoxyethanol, und aromatische Aldehyde in einem bestimmten Mengenverhältnis eingesetzt werden.
Aus der US-A-3 506 990 ist ein Verfahren zum Färben von aromatischen Polyamidfasern bekannt, bei dem als Färbehilfsstoff eine Vielzahl strukturell verschiedener Verbindungen verwendet werden kann, unter anderem Benzylalkohol oder Salicylaldehyd.

In dem Artikel von Zoltan Csuros und Andor Lorinc in Przegl. Wlok. (1967), 21(2), 90-1, wird die Verwendung von m-Kresol bei der Färbung von Polyesterfasern beschrieben.

Das Aufziehverhalten von sauren Farbstoffe kann zwar durch den Zusatz von sogenannten Carriem oder Penetrationsbeschleunigern, wie zum Beispiel Benzylalkohol oder 2-Benzyloxyethanol, verbessert werden, gleichzeitig wird hierdurch jedoch auch das Ausmaß der Hautanfärbung verstärkt. Zudem ist ein Teil der bekannten Carrier auch in toxikologischer Hinsicht nicht ganz unbedenklich.

Es bestand daher die Aufgabe, ein Färbemittel, insbesondere zur Färbung von Haaren, auf der Basis von sauren Farbstoffen zur Verfügung zu stellen, das eine intensive und gleichmäßige Färbung der Fasern gewährleistet und gleichzeitig keine oder nur eine geringe Anfärbung der Haut bedingt und toxikologisch unbedenklich ist.

Überraschenderweise wurde nunmehr gefunden, daß bei Verwendung von bestimmten Carriem eine intensive und gleichmäßigere Anfärbung der Fasern erzielt werden kann, wobei insbesondere das Aufziehverhalten von im langwelligen Bereich (λ > 500 mm) absorbierenden Farbstoffmolekülen deutlich verbessert wird.

Gegenstand der vorliegenden Erfindung ist daher ein Mittel zum Färben von natürlichen oder synthetischen Fasern, wie zum Beispiel Baumwolle, Wolle, Seide, Viskose, Nylon, Celluloseacetat und insbesondere Keratinfasem wie beispielsweise menschlichen Haaren, welches
(i) mindestens einen sauren Farbstoff sowie
(ii) mindestens einen Carrier, welcher ausgewählt ist aus Vanillin (4-Hydroxy-3-methoxy-benzaldehyd), p-Hydroxyanisol, 3-Hydroxy-4-methoxy-benzahldehyd, 3,5-Dihydroxybenzaldehyd, 3,4-Dihydroxybenzaldehyd, 4-Hydroxy-phenylacetamid, p-Hydroxybenzaldehyd, Hydrochinonmonomethylether, o-Fluorphhenol, m-Fluorphenol, p-Fluorphenol, 2-(2'-Hydroxyphenoxy)-ethanol, 3,4-Methylendioxy-phenol, Resorcinmonomethylether, 3,4-Dimethoxy-phenol, 3-Trifluormethylphenol, Resorcinmonoacetat, Ethylvanillin und 2-Thiophenethanol, enthält. Vanillin, alleine oder in Kombination mit anderen Carriem, ist hierbei besonders bevorzugt.
Der Carrier wird vorzugsweise in einer Menge von 0,1 bis 20 Gewichtsprozent, insbesondere 1 bis 9 Gewichtsprozent, eingesetzt.

Besonders intensive Färbungen werden bei Verwendung von sauer eingestellten Färbemitteln mit einem pH-Wert von 1,5 bis 5,0, insbesondere 2,5 bis 3,5, erhalten.

Für die Einstellung des erfindungsgemäßen sauren pH-Wertes sind insbesondere die folgenden Säuren geeignet: α-Hydroxy-carbonsäuren, wie zum Beispiel Glykolsäure, Milchsäure, Weinsäure, Zitronensäure oder Apfelsäure; Ascorbinsäure; Gluconsäurelacton; Essigsäure und Phosphorsäure; sowie Mischungen der genannten Säuren, wobei die Verwendung von Milchsäure und Glykolsäure besonders bevorzugt ist. Die Einsatzmenge der vorgenannten Säuren liegt in der Regel bei 0,1 bis 10 Gewichtsprozent, vorzugsweise 1 bis 2 Gewichtsprozent.

Als erfindungsgemäße Farbstoffe können saure Azofarbstoffe, Nitrofarbstoffe, Anthrachinonfarbstoffe oder Chinolinfarbstoffe verwendet werden. Als Beispiel für geeignete Farbstoffe können insbesondere die folgenden im CTFA-International Cosmetic Ingredient Dictionary genannten Farbstoffe genannt werden:
2,4-Dinitro-1-naphthol-7-sulfonsäure-Dinatriumsalz (Acid Yellow 1; CI 10 316);
2-(2'-Chinolyl)-1H-indene-1,3(2H)-dion-monodisulfonsäure-Dinatriumsalz (Acid Yellow 3; CI 47 005);
4,5-Dihydro-5-oxo-1-(4'-sulfophenyl)-4-[(4''-sulfophenyl) azo]-1H-pyrazol-3-carbonsäure-Trinatriumsalz (Acid Yellow 23; CI 19 140);
3',6'-Dihydroxyspiro [isobenzofuran-1(3H), 9' (9H)-xanthen]-3-on (Acid Yellow 73; CI 45 350:1);
5-[2',4'-Dinitrophenyl)amino]-2-(phenylamino)-benzolsulfonsäure-Natriumsalz (Acid Orange 3; CI 10 385);
4-[(2',4' -Dihydroxyphenyl)azo]-benzolsulfonsäure-Natrium-salz (Acid Orange 6; CI 14 270);
4-[2'-Hydroxy-1'-naphthyl)azo]-benzolsulfonsäure-Natriumsalz (Acid Orange 7; CI 15 510);
4-[[3'-[(2'',4''-Dimethylphenyl)azo]-2',4'-dihydroxyphenyl]azo]-benzolsulfonsäure-Natriumsalz (Acid Orange 24;CI 20 170);
4-Hydroxy-3-[(4'-sulfo-1'-naphthyl)azo]-1-naphthalinsulfonsäure-Dinatriumsalz (Acid Red 14; CI 14 720);
7-Hydroxy-8-[(4'-sulfo-1'-naphthyl)azo]-1,3-naphthalindisulfonsäure-Trinatriumsalz (Acid Red 18; CI 16 255);
3-Hydroxy-4-[(4'-sulfo-1'-naphthyl)azo]-2,7-naphthalindisulfonsäure-Trinatriumsalz (Acid Red 27; CI 16 185);
5-Amino-4-hydroxy-3-phenylazo-2,7-naphtalindisulfonsäure-Dinatriumsalz (Acid Red 33; CI 17 200);
5-(Acetylamino)-4-hydroxy-3-[(2'-methylphenyl)azo]-2,7-naphthalindisulfonsäure-Dinatriumsalz (Acid Red 35; CI 18 065);
3',6'-Dihydroxy-2',4',5',7'-tetraiodospiro-[isobenzofuran-1 (3H), 9' (9H)-xanthen]-3-on-Dinatriumsalz (Acid Red 51; CI 45 430);
3,6-Bis-(diethylamino)-9-(2',4'-disulfophenyl)-xanthyliumhydroxid-Natriumslz (Acid Red 52; CI 45 100);
7-Hydroxy-8-[[4'-(phenylazo) phenyl]azo]-1,3-naphthalindisulfonsäure-Dinatriumsalz (Acid Red 73; CI 27 290);
2',4',5',7'-Tetrabromo-3',6'-dihydroxyspiro[isobenzofuran-1(3H),9'(9H)-xanthen]-3-on-Dinatriumsalz (Acid Red 87; CI 45 380);
2',4',5',7'-Tetrabromo-4,5,6,7-tetrachloro-3',6'-dihydroxyspiro[isobenzofuran-1(3H) , 9' (9H)-xanthen]-3-on-Dinatriumsalz (Acid Red 92; CI 45 410);
3',6'-Dihydroxy-4',5'-dijodospiro[isobenzofuran-1(3H),9'(9H)-xanthen]-3-on-Dinatriumsalz (Acid Red 95; CI 45 425);
Acid Red 195; Acid Blue 9 (CI 42 090);
2,2'-[(9,10-Dihydro-9,10-dioxo-1,4-anthracendiyl)-diimino]-bis-(5-methyl-benzolsulfonsäue)-Dinatriumsalz (Acid Green 25; CI 61 570);
N-[4-[[4'-(Dimethylamino) phenyl]- (2''-hydroxy-3'',6''-disulfo-1''-naphthyl)-methylen]-2,5-cyclohexadien-1-yliden]-N-methylmethanaminiumhydroxid (Acid Green 50; CI 44 090);
N-[4-[[4'-Diethylamino)phenyl]-(2'',4''-disulfophenyl)-methylen]-2,5-cyclohexadien-1-yliden]-N-ethylethanaminiumhydroxid-Natriumsalz (Acid Blue 1; CI 42 045);
N-[4-[[4'-Diethylamino) phenyl]-(5''-hydroxy-2'',4''-disulfo-phenyl)-methylen]-2,5-caclohexadien-1-yliden]-N-ethyl-ethanaminiumhydroxid-Calciumsalz (Acid Blue 3;
CI 42 051);
1-Amino-4-(cyclohexylamino)-9,10-dihydro-9,10-dioxo-2-anthracensulfonsäure-Natriumsalz (Acid Blue 62; CI 62 045);
2-(1',3'-Dihydro-3'-oxo-5'-sulfo-2'H-indol-2'-yliden)-2,3-dihydro-3-oxo-1H-indol-5-sulfonsäure-Dinatriumsalz (Acid Blue 74; CI 73 015);
9-(2'-Carboxyphenyl)-3-[(2''-methylphenyl)amino]-6-[(2'''-methyl-4'''-sulfophenyl)amino)]-xanthyliumhydroxid-Natriumsalz (Acid Violet 9; CI 45 190);

2-[(9',10'-Dihydro-4'-hydroxy-9',10'-dioxo-1'-anthracenyl)-amino]-5-methylbenzolsulfonsäure-Natriumsalz (Acid Violet 43; CI 60 730);
3, 3'-[Sulfonyl-bis(2-nitro-4,1-phenylen)imino]-bis-[6-phenylamino)-benzol-dinatriumsulfonat] (Acid Brown 13; CI 10 410);
4-Amino-5-hydroxy-3-[(4'-nitrophenyl)azo]-6-(phenylazo)-2,7-naphthalindisulfonsäure-Dinatriumsalz (Acid Black 1; CI 20 470);
3-Hydroxy-4-[(2'-hydroxy-1'-naphthyl)azo]-7-nitro-1-naphthalinsulfonsäure-Natriumsalz (Acid Black 52; CI 15 711);
4-(Acetylamino)-5-hydroxy-6-[[7'-sulfo-4'-[(4''-Sulfophenyl)azo]-1'-naphthyl]azo]-1,7-naphthalin-disulfonsäure-Tetranatriumsalz (Brilliant Black 1; CI 28 440).

Die vorgenannten Farbstoffe können, falls erforderlich, auch in Kombination mit nicht-ionischen oder basischen Farbstoffen eingesetzt werden.

Der Gesamtgehalt an Farbstoffen beträgt in dem erfindungsgemäßen Färbemittel vorzugsweise 0,01 bis 5 Gewichtsprozent.

Das erfindungsgemäße Färbemittel kann weiterhin alle für derartige Zubereitungen bekannten und üblichen Zusatzstoffe, beispielsweise Parfümöle; Komplexbildner; Wachse; Konservierungsstoffe, kosmetische Harze, wie zum Beispiel Polyvinylpyrrolidon oder Polyvinylacetat; Verdicker; Alginate; Guar Gum; haarpflegende Substanzen, wie zum Beispiel kationische Polymere oder Lanolinderivate; oder Netzmittel und Emulgatoren aus den Klassen der anionischen, nichtionischen, amphoteren oder kationischen oberflächenaktiven Substanzen, enthalten.

Als besonders günstig hat sich hierbei der Zusatz von nicht-ionischen und/oder amphoteren Tensiden erwiesen.

Die erwähnten Bestandteile werden in den für solche Zwecke üblichen Mengen verwendet, zum Beispiel die Netzmittel und Emulgatoren in einer Konzentration von 0,1 bis 30 Gewichtsprozent, vorzugsweise 0,1 bis 10 Gewichtsprozent, und die Pflegestoffe in einer Menge von 0,1 bis 5 Gewichtsprozent.

Das erfindungsgemäße Färbemittel kann neben Wasser weitere Lösungsmittel, wie zum Beispiel aliphatische Alkohole, insbesondere Ethanol oder Isopropanol, oder Glykolether, insbesondere 1,2-Propandiol, enthalten, wobei der Wassergehalt in der Regel etwa 25 bis 95 Gewichtsprozent, vorzugsweise 30 bis 85 Gewichtsprozent, beträgt, während der Gehalt an den übrigen Lösungsmitteln bei etwa 5 bis 30 Gewichtsprozent liegt.

Das erfindungsgemäße Färbemittel kann in Form einer wäßrigen oder wäßrig-alkoholischen Lösung, einer Creme, eines Geles, einer Emulsion oder eines Aerosolschaumes vorliegen, wobei das Färbemittel sowohl in Form eines Einkomponentenpräparates als auch in Form eines Mehrkomponentenpräparates konfektioniert sein kann.

Die Anwendung des erfindungsgemäßen Färbemittels erfolgt im Falle der Haarfärbung in bekannter Weise indem man eine für die Haarfärbung ausreichende Menge, je nach Haarlänge etwa 30 bis 120 Gramm, des Färbemittels auf das Haar aufträgt, das Färbemittel bei 15 bis 50 Grad Celsius etwa 5 bis 60 Minuten, insbesondere 15 bis 30 Minuten, einwirken läßt, und sodann das Haar gründlich mit Wasser ausspült und trocknet.

Das erfindungsgemäße Haarfärbemittel ermöglicht eine hervorragende, gleichmäßige und intensive Färbung der Haare ohne nennenswerte Anfärbung der Kopfhaut.

Die Intensität der Färbungen kann bei der Färbung von temperaturstabilen Fasern (beispielsweise Baumwolle oder bestimmten synthetischen Fasern) durch eine Erhöhung der Behandlungstemperatur auf bis zu 100 Grad Celsius zusätzlich gesteigert werden.

Es zeigt sich weiterhin, daß die Intensität der Färbungen umso deutlicher durch den Carrier gesteigert wird, je langwelliger der verwendete Farbstoff absorbiert.

Die nachfolgenden Beispiele sollen den Gegenstand der vorliegenden Erfindung näher erläutern, ohne diesen hierauf zu beschränken.

### Beispiele

### Beispiel 1: Haarfärbemittel:

| | |
|---|---|
| 2,1 g | Natrium-cocoamphoacetat(40%ige wäßrige Lösung) |
| 1,3 g | Glykolsäure |
| 5,0 g | Isopropanol |
| 2,0 g | 1,2-Propandiol |
| 0,3 g | di-Natrium-EDTA |
| 4,0 g | Vanillin |
| 1,5 g | Acid-Red 14 (C.I. 14 720) |
| 83,8 g | Wasser, vollentsalzt |
| 100,0 g | |

Die Mischung wird auf gebleichte Haarsträhnen aufgetragen. Nach 20-minütiger Einwirkzeit bei Raumtemperatur werden die Haare gewaschen und getrocknet.

Es wird ein rosa-roter Farbton (L = 40,27; a = 60,49; b = 9,06) erhalten.

Bei Verwendung eines Mittels gemäß Beispiel 1, in dem das Vanillin mengengleich durch Wasser ersetzt wurde, wird eine deutlich schwächere Färbung erhalten (L = 44,65; a = 56,03; b = 6,10).

### Beispiel 2: Färbemittel:

| | |
|---|---|
| 2,1 g | Natrium Cocoamphoacetat (40%ige wäßrige Lösung) |
| 1,3 g | Glykolsäure |
| 5,0 g | Isopropanol |
| 2,0 g | 1,2-Propandiol |
| 0,3 g | di-Natrium-EDTA |
| 4,0 g | Vanillin |
| 2,2 g | Brillantblack 1 (C.I. 28 440) |
| 83,1 g | Wasser, vollentsalzt |
| 100,0 g | |

Die Mischung wird auf gebleichte Haarsträhnen aufgetragen. Nach 20-minütiger Einwirkzeit bei Raumtemperatur werden die Haare gewaschen und getrocknet. Es wird ein intensiver blauer Farbton (L = 42,43; a = 3,73; b = -17,07) erhalten.

Bei Verwendung eines Mittels gemäß Beispiel 2, in dem das Vanillin mengengleich durch Wasser ersetzt wurde, wird eine deutlich schwächere Färbung erhalten (L = 55,65; a = 3,63; b = -10,08).

### Beispiel 3 bis 11: Färbemittel:

| | |
|---|---|
| 2,00 g | Laurylpolyglucose (50%ig) |
| | (Plantaren 1200 CS/UP) |
| 1,30 g | Glykolsäure |
| 5,00 g | Isopropanol |
| 2,00 g | 1,2-Propandiol |
| 0,20 g | di-Natrium-EDTA |
| 0,04 g | Acid Yellow 1 (C.I. 10 316) |
| 0,54 g | Acid Orange 7 (C.I. 15 510) |
| 0,46 g | Acid Red 18 (C.I. 16 255) |
| 0,29 g | Acid Black 1 (C.I. 20 470) |
| 0,22 g | Acid Violet 43 (C.I. 60 730) |
| 4,00 bis 9,00 g | Carrier gemäß Tabelle 1 |
| ad 100 g | Wasser vollentsalzt |

Die Färbemittel 3 bis 13 werden auf gebleichte Büffelhaar-Strähnen aufgetragen. Nach 20minütiger Einwirkzeit bei 40 °C und anschließendem Waschen erhält man orange-braune bis tief dunkelbraune Färbungen (vergleiche die nachfolgende Tabelle 1).

**Tabelle 1**

| Beispiel | Carrier | Menge an Carrier | log P des verwendeten Carriers | L | a | b |
|---|---|---|---|---|---|---|
| | | | | Wert | | |
| Vergleich | ohne (ersetzt durch Wasser) | 0 % | - | 27,53 | +14,48 | +15,42 |
| 3 | Vanillin | 4 % | 1,00 | 20,81 | +6,78 | +5,39 |
| 4 | Hydrochinonmonomethylether | 4% | 1,55 | 21,07 | +8,89 | +6,84 |
| | | 9 % | | 18,64 | +3,41 | +1,54 |
| 5 | 4-Hydroxy-benzaldehyd | 4 % | 0,98 | 22,07 | +7,33 | +5,82 |
| 6 | Resorcin-monomethylether | 4 % | 1,55 | 23,01 | +8,18 | +6,76 |
| | | 9 % | | 17,96 | +2,95 | +1,02 |
| 7 | 2-Fluorphenol | 4 % | 1,69 | 23,39 | +9,90 | +8,67 |
| | | 9% | | 18,57 | +4,43 | +1,61 |
| 8 | 3-Fluorphenol | 4 % | 1,69 | 23,16 | +10,41 | +7,91 |
| | | 9% | | 19.88 | +5,57 | +2,69 |
| 9 | 4-Fluorphenol | 4 % | 1,69 | 22,20 | +9,41 | +7,19 |
| | | 9 % | | 17,99 | +3,63 | +0,97 |
| 10 | 3,4-Methylendioxo-phenol | 4 % | 1,32 | 23,32 | +9,10 | +8,05 |
| 11 | Resorcinol-monoacetat | 4 % | 1,04 | 23,61 | +8.73 | +8,18 |

Die in der vorliegenden Anmeldung verwendeten Prozentangaben stellen, soweit nicht anders vermerkt, Gewichtsprozente dar. Die Ermittlung der Lab-Farbmeßwerte erfolgte mit einem Farbmeßgerät der Firma Minolta, Typ Chromameter II.

Hierbei steht der L-Wert für die Helligkeit (das heißt, je geringer der L-Wert ist, um so größer ist die Farbintensität), während der a-Wert ein Maß für den Rotanteil ist (je größer der a-Wert ist, um so höher ist der Rotanteil). Der b-Wert ist ein Maß für den Blauanteil der Farbe, wobei der Blauanteil um so größer ist, je negativer der b-Wert ist.

## Patentansprüche

1. Mittel zum Färben von Fasern, welches **dadurch gekennzeichnet ist, daß** es (i) mindestens einen sauren Farbstoff sowie (ii) mindestens einen Carrier weicher ausgewählt ist aus Vanillin, p-Hydroxyanisol, 3-Hydroxy-4-methoxy-benzaldehyd, 3,5-Dihydroxybenzaldehyd, 3,4-Dihydroxy-benzaldehyd, 4-Hydroxy-phenylacetamid, p-Hydroxy-benzaldehyd, Hydrochinon-monomethylether, o-Fluor-phenol, m-Fluorphenol, p-Fluorphenol, 2-(2'-Hydroxyphenoxy)-ethanol, 3,4-Methylendioxy-phenol, Resorcinmonomethylether, 3,4-Dimethoxy-phenol, 3-Trifluor-methyl-phenol, Resorcinmonoacetat, Ethylvanillin und 2-Thiophenethanol, enthält.

2. Mittel nach Anspruch 1, **dadurch gekennzeichnet, daß** der Carrier in einer Menge von 0,1 bis 20 Gewichtsprozent eingesetzt wird.

3. Mittel nach Anspruch 1 oder 2, **dadurch gekennzeichnet, daß** es einen pH-Wert von 1,5 bis 5 aufweist.

4. Mittel nach einem der Ansprüche 1 bis 3, **dadurch gekennzeichnet, daß** es 0,1 bis 10 Gewichtsprozent mindestens einer Säure enthält.

5. Mittel nach einem der Ansprüche 1 bis 4, **dadurch gekennzeichnet, daß** die Säure ausgewählt ist aus Glykolsäure, Milchsäure, Weinsäure, Zitronensäure, Äpfelsäure, Ascorbinsäure, Gluconsäurelacton, Essigsäure und Phosphorsäure, sowie Mischungen der genannten Säuren.

6. Mittel nach einem der Ansprüche 1 bis 5, **dadurch gekennzeichnet, daß** der saure Farbstoff ausgewählt ist aus sauren Azofarbstoffen, Nitrofarbstoffen, Anthrachinonfarbstoffen und Chinolinfarbstoffen.

7. Mittel nach einem der Ansprüche 1 bis 6, **dadurch gekennzeichnet, daß** der Farbstoff ausgewählt ist aus Acid Yellow 1 (C.I. 10 316), Acid Yellow 3(C.I. 47 005), Acid Yellow 23 (C.I. 19 140),Acid Yellow 73 (C.I. 45 350:1), Acid Orange 3(C.I. 10 385), Acid Orange 6 (C.I. 14 270), Acid Orange 7 (C.I. 15 510), Acid Orange 24 (C.I. 20 170), Acid Red 14 (C.I. 14 720), Acid Red 18 (C.I. 16 255), Acid Red 27 (C.I. 16 185), Acid Red 33 (C.I. 17 200), Acid Red 35 (C.I. 18 065), Acid Red 51 (C.I. 45 430), Acid Red 52 (C.I. 45 100), Acid Red 73 (C.I. 27 290), Acid Red 87 (C.I. 45 380), Acid Red 92 (C.I. 45 410), Acid Red 95 (C.I. 45 425), Acid Red 195, Acid Blue 9 (C.I. 42 090); Acid Green 25 (C.I. 61 570), Acid Green 50 (C.I. 44 090), Acid Blue 1 (C.I. 42 045), Acid Blue 3 (C.I. 42 051), Acid Blue 62 (C.I. 62 045), Acid Blue 74 (C.I. 73 015), Acid Violet 9 (C.I. 45 190), Acid Violet 43 (C.I. 60 730), Acid Brown 13 (C.I. 10 410), Acid Black 1 (C.I. 20 470), Acid Black 52 (C.I. 15 711) und Brilliant Black 1 (C.I. 28 440) oder Mischungen der vorgenannten Farbstoffe.

8. Mittel nach einem der Ansprüche 1 bis 7, **dadurch gekennzeichnet, daß** der saure Farbstoff in einer Gesamtmenge von 0,01 bis 5 Gewichtsprozent enthalten ist.

9. Mittel nach einem der Ansprüche 1 bis 8, **dadurch gekennzeichnet, daß** es 0,1 bis 10 Gewichtsprozent mindestens einer nicht-ionischen und/oder amphoteren oberflächenaktiven Substanz enthält.

10. Mittel nach einem der Ansprüche 1 bis 9, **dadurch gekennzeichnet, daß** es ein Haarfärbemittel ist.

11. Verfahren zum Färben von Haaren, **dadurch gekennzeichnet, daß** ein Färbemittel gemäß einem der Ansprüche 1 bis 10 in einer Menge von 30 bis 120 g auf die Haare aufgetragen wird, und das Haar nach einer Einwirkungszeit von 5 bis 60 Minuten bei 15 bis 50 °C mit Wasser ausgespült und getrocknet wird.

## Claims

1. Agent for colouring fibres, which is **characterized in that** it comprises (i) at least one acidic dye and (ii) at least one carrier which is selected from vanillin, p-hydroxyanisole, 3-hydroxy-4-methoxybenzaldehyde, 3,5-dihydroxybenzaldehyde, 3,4-dihydroxybenzaldehyde, 4-hydroxyphenylacetamide, p-hydroxybenzaldehyde, hydroquinone monomethyl ether, o-fluorophenol, m-fluorophenol, p-fluorophenol, 2-(2'-hydroxy-phenoxy)ethanol, 3,4-methylenedioxyphenol, resorcinol monomethyl ether, 3,4-dimethoxyphenol, 3-trifluoromethylphenol, resorcinol monoacetate, ethylvanillin and 2-thiopheneethanol.

2. Agent according to Claim 1, **characterized in that** the carrier is used in an amount of from 0.1 to 20% by weight.

3. Agent according to Claim 1 or 2, **characterized in that** it has a pH of from 1.5 to 5.

4. Agent according to one of Claims 1 to 3, **characterized in that** it comprises 0.1 to 10% by weight of at least one acid.

5. Agent according to one of Claims 1 to 4, **characterized in that** the acid is chosen from glycolic acid, lactic acid, tartaric acid, citric acid, malic acid, ascorbic acid, gluconolactone, acetic acid and phosphoric acid, and mixtures of said acids.

6. Agent according to one of Claims 1 to 5, **characterized in that** the acidic dye is selected from acidic azo dyes, nitro dyes, anthraquinone dyes and quinoline dyes.

7. Agent according to one of Claims 1 to 6, **characterized in that** the dye is selected from Acid Yellow 1 (C. I. 10 316), Acid Yellow 3 (C.I. 47 005), Acid Yellow 23 (C.I. 19 140), Acid Yellow 73 (C.I. 45 350:1), Acid Orange 3 (C.I. 10 385), Acid Orange 6 (C.I. 14 270), Acid Orange 7 (C.I. 15 510), Acid Orange 24 (C.I. 20 170), Acid Red 14 (C.I. 14 720), Acid Red 18 (C. I. 16 255), Acid Red 27 (C.I. 16 185), Acid Red 33 (C.I. 17 200), Acid Red 35 (C.I. 18 065), Acid Red 51 (C.I. 45 430), Acid Red 52 (C.I. 45 100), Acid Red 73 (C.I. 27 290), Acid Red 87 (C.I. 45 380), Acid Red 92 (C.I. 45 410), Acid Red 95 (C.I. 45 425), Acid Red 195, Acid Blue 9 (C.I. 42 090) ; Acid Green 25 (C.I. 61 570), Acid Green 50 (C.I. 44 090), Acid Blue 1 (C.I. 42 045), Acid Blue 3 (C.I. 42 051), Acid Blue 62 (C.I. 62 045), Acid Blue 74 (C.I. 73 015), Acid Violet 9 (C.I. 45 190), Acid Violet 43 (C.I. 60 730), Acid Brown 13 (C.I. 10 410), Acid Black 1 (C.I. 20 470), Acid Black 52 (C.I. 15 711) and Brilliant Black 1 (C.I. 28 440), or mixtures of the abovementioned dyes.

8. Agent according to one of Claims 1 to 7, **characterized in that** the acidic dye is present in a total amount of from 0.01 to 5% by weight.

9. Agent according to one of Claims 1 to 8, **characterized in that** it comprises 0.1 to 10% by weight of at least one nonionic and/or amphoteric surface-active substance.

10. Agent according to one of Claims 1 to 9, **characterized in that** it is a hair colorant.

11. Method of colouring hair, **characterized in that** a colorant according to one of Claims 1 to 10 is applied to the hair in an amount of from 30 to 120 g, and, after a contact time of from 5 to 60 minutes at 15 to 50°C, the hair is rinsed with water and dried.

## Revendications

1. Agent pour la teinture de fibres, **caractérisé en ce qu'**il contient (i) au moins un colorant acide ainsi que (ii) au moins un support choisi parmi la vanilline, le p-hydroxyanisole, le 3-hydroxy-4-méthoxybenzaldéhyde, le 3,5-dihydroxybenzaldéhyde, le 3,4-dihydroxybenzaldéhyde, le 4-hydroxyphénylacétamide, le p-hydroxybenzaldéhyde, l'hydroquinonemonométhyléther, l'o-fluorophénol, le m-fluorophénol, le p-fluorophénol, le 2-(2'-hydroxyphénoxy)éthanol, le 3,4-méthylènedioxyphénol, le résorcinolmonométhyléther, le 3,4-diméthoxyphénol, le 3-trifluorométhylphénol, le monoacétate de résorcinol, l'éthylvanilline et le 2-thiophénéthanol.

2. Agent selon la revendication 1, **caractérisé en ce que** le support est utilisé en une quantité de 0,1 à 20% en poids.

3. Agent selon la revendication 1 ou 2, **caractérisé en ce qu'**il présente un pH de 1,5 à 5.

4. Agent selon l'une quelconque des revendications 1 à 3, **caractérisé en ce qu'**il contient 0,1 à 10% en poids d'au moins un acide.

5. Agent selon l'une quelconque des revendications 1 à 4, **caractérisé en ce que** l'acide est choisi parmi l'acide glycolique, l'acide lactique, l'acide tartrique, l'acide citrique, l'acide malique, l'acide ascorbique, la lactone de l'acide gluconique, l'acide acétique et l'acide phosphorique, ainsi que des mélanges des acides mentionnés.

6. Agent selon l'une quelconque des revendications 1 à 5, **caractérisé en ce que** le colorant acide est choisi parmi les colorants acides azo, nitro, anthraquinone et quinoléine.

7. Agent selon l'une quelconque des revendications 1 à 6, **caractérisé en ce que** le colorant est choisi parmi l'Acid Yellow 1 (C.I. 10 316), l'Acid Yellow 3 (C.I. 47 005), l'Acid Yellow 23 (C.I. 19 140), l'Acid Yellow 73 (C.I. 45 350:1), l'Acid Orange 3 (C.I. 10 385), l'Acid Orange 6 (C.I. 14 270), l'Acid Orange 7 (C.I. 15 510), l'Acid Orange 24 (C.I. 20 170), l'Acid Red 14 (C.I. 14 720), l'Acid Red 18 (C.I. 16 255), l'Acid Red 27 (C.I. 16 185), l'Acid Red 33 (C.I. 17 200), l'Acid Red 35 (C.I. 18 065), l'Acid Red 51 (C.I. 45 430), l'Acid Red 52 (C.I. 45 100), l'Acid Red 73 (C.I. 27 290), l'Acid Red 87 (C.I. 45 380), l'Acid Red 92 (C.I. 45 410), l'Acid Red 95 (C.I. 45 425), l'Acid Red 195, l'Acid Blue 9 (C.I. 42 090), l'Acid Green 25 (C.I. 61 570), l'Acid Green 50 (C.I. 44 090), l'Acid Blue 1 (C.I. 42 045), l'Acid Blue 3 (C.I. 42 051), l'Acid Blue 62 (C.I. 62 045), l'Acid Blue 74 (C.I. 73 015), l'Acid Violet 9 (C.I. 45 190), l'Acid Violet 43 (C.I. 60 730), l'Acid Brown 13 (C.I. 10 410), l'Acid Black 1 (C.I. 20 470), l'Acid Black 52 (C.I. 15 711) et le Brilliant Black 1 (C.I. 28 440), ou des mélanges des colorants susmentionnés.

8. Agent selon l'une quelconque des revendications 1 à 7, **caractérisé en ce que** le colorant acide est contenu en une quantité totale de 0,01 à 5% en poids.

9. Agent selon l'une quelconque des revendications 1 à 8, **caractérisé en ce qu'**il contient 0,1 à 10% en poids d'au moins une substance tensioactive non ionique et/ou amphotère.

10. Agent selon l'une quelconque des revendications 1 à 9, **caractérisé en ce qu'**il s'agit d'un agent de teinture pour cheveux.

11. Procédé pour la teinture de cheveux, **caractérisé en ce qu'**on applique un agent de teinture selon l'une quelconque des revendications 1 à 10 en une quantité de 30 à 120 g sur les cheveux et les cheveux sont rincés avec de l'eau et séchés après un temps d'action de 5 à 60 minutes à 15 jusqu'à 50°C.
